# EUROPEAN PATENT APPLICATION

(11) **EP 1 192 940 A1**
(43) Date of publication of application: **03.04.2002**
(21) Application number: 01308360.5
(22) Date of filing: 01.10.2001
(51) Int. Cl.: A61K 7/48

(54) **Compositions and methods for promoting clear skin using an alkanolamine**

(30) Priority: 02.10.2000 US 677738
(71) Applicant: Johnson & Johnson Consumer Companies, Inc., Skillman, NJ 08558-9418 (US)
(72) Inventor: Cole, Curtis, Ringoes, NJ 08551 (US); Ganopolsky, Irina, Lawrenceville, NJ 08648 (US); Lukenbach, Elvin, Flemington, NJ 08822 (US)
(74) Representative: Mercer, Christopher Paul

(57) **Abstract**

The invention relates to a method for reducing the number and severity of acne papules on mammalian skin comprising topically applying a composition comprising an effective amount of an alkanolamine in a cosmetically acceptable carrier. The alkanolamine has the following general formula: 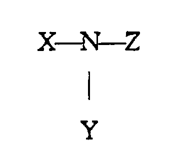 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.
The compositions used in the method according to the skin are well-tolerated by the skin and are safe for continued use over a long time period

## Description

### 1. Field of the Invention

This invention relates to compositions and methods for promoting clear skin in subjects with acne or acne-prone skin. More particularly, it relates to compositions containing at least one alkanolamine and their application to mammalian skin to effect a reduction in acne papules in human skin.

### 2. Background of the Invention

Acne is an inflammatory dermatological disorder which occurs frequently in adolescence and with some regularity in older adults of the human species. The condition of acne can include skin lesions ranging from the comedo or "blackhead" in a pilosebaceous follicle, to more severe symptioms such as pustules, papules, cysts and nodules. The condition is not only uncomfortable for the victim, but embarrassing, and can result in disfigurement and scarring.

Many different approaches to ameliorating this disorder have been attempted in the past, with some treatments more effective than others. Attacks ranging from simple washing and cleansing to pharmaceuticals have been employed.

It is desired to have a single topical treatment that could prevent or reverse acne which does not require a pharmaceutical prescription. It is more desired to have a single and affordable topical treatment for daily skin care, which will provide these benefits, with no irritation or negative side effects, and which will further provide the desired homogeneous skin complexion, even out skin texture and tone, and induce skin firming and a healthier look

Thus, it is an object of this invention to provide topical compositions that can be used to ameliorate acne papules in mammalian skin.

It is another object of this invention is to provide topical compositions to ameliorate acne papules and that are well-tolerated by the skin.

Yet another object of this invention is to provide a method of ameliorating acne papules using a topically-applied composition.

Still another object of this invention is to provide a method of ameliorating acne papules and which can additionally relieve symptoms of acne such as redness and inflammation.

Yet another object of this invention is to provide a composition capable of relieving acne which is safe for continued use over a long time period.

### Summary of the Invention

It has been discovered that topical compositions comprising an effective amount of an alkanolamine can be used to safely and effectively ameliorate acne papules in mammalian skin. Accordingly, the invention relates to a method for reducing the number and severity of acne papules on mammalian skin comprising topically applying a composition comprising an effective amount of an alkanolamine of the formula: 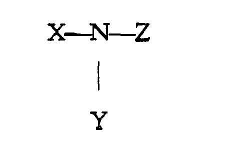 in a cosmetically acceptable carrier. In the formula X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.

Compositions comprising an alkanolamine as described above have also been found to reduce redness caused by acne lesions within one hour of application (see co-pending European patent application No. , entitled "Methods for Reduction of Inflammation and Erythema" concurrently filed herewith), Attorney's ref: P028248EP.

### Brief Description of the Drawings

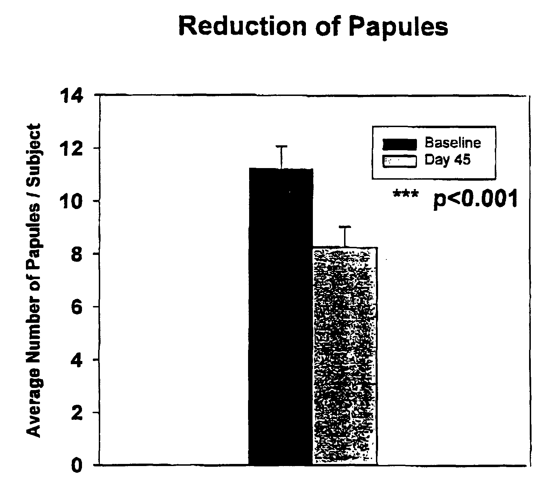

Figure 1 is a bar graph showing the reduction of papules on subjects when a composition according to the invention is topically applied to the skin as described in Example 2.

### Detailed Description of the Preferred Embodiments

As described above, the invention relates to a method for reducing the number and severity of acne papules on mammalian skin comprising topically applying a composition comprising an effective amount of an alkanolamine and a cosmetically acceptable carrier. The alkanolamine used in the methods according to the invention has the following general formula: 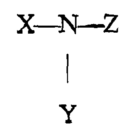 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.

In a preferred embodiment the alkanolamine is selected from the group consisting of ethylaminoethanol, methylaminoethanol, dimethylaminoethanolamine, isopropanolamine, triethanolamine, isopropanoldimethylamine, ethylethanolamine, 2-butanolamine, choline and serine. More preferably, the alkanolamine is dimethylaminoethanol (DMAE).

The compositions used in the methods according to the invention preferably contain from about 0.1 about 10% by weight of the alkanolamine, more preferably, from about 0.1 to about 5% and, most preferably, from about 1 to about 3% by weight based on the total composition.

In a preferred embodiment, the compositions used in the methods of the invention contain a pH buffering agent. Preferably, the amount of buffering agent should be that which would result in compositions having a pH ranging from about 4.5 to about 8.5, more preferably from about 5.5 to about 8.5, most preferably from about 6.5 to about 8.0. The buffering agent can be any of the known buffering agents commonly found in cosmetic compositions provided that they are physically and chemically stable with the other ingredients of the composition. Suitable buffering agents include but are not intended to be restricted to organic acids such as citric acid, malic acid, and glycolic acid.

Another compound which is advantageously present in the compositions of this invention is tyrosine. Tyrosine may be present in the compositions of this invention in the amount of from about 0.01 to about 5%, more preferably from about 0.04 to about 3% by weight and most preferably about 0.5% by weight, based on the total composition.

It may be advantageous to also incorporate other acne fighting ingredients to also fight acne papules and other acne lesions such as comedones and pustules. Such well recognized ingredients include salicylic acid, benzyl peroxide, sulfur, resorsinol, retinoids (such as but not limited to retinol, retinal, retinyl palmitate, retinoic acid). The combination of these ingredients with the alkanolamines are anticipated to have additive effects if not synergistic due to different mechanisms of action.

The compositions of this invention should be in the form of topical products that can be applied externally to the skin and can be prepared in accordance with conventional techniques known to those of ordinary skill in the art. The carrier may take a variety of physical forms such as, for example, creams, dressings, gels, lotions, ointments or liquids.

Typical carriers include lotions containing water and/or alcohols and emollients such as hydrocarbon oils and waxes, silicone oils, hyaluronic acid, vegetable, animal or marine fats or oils, glyceride derivatives, fatty acids or fatty acid esters or alcohols or alcohol ethers, lanolin and derivatives, polyhydric alcohols or esters, wax esters, sterols, phospholipids and the like, and generally also emulsifiers (nonionic, cationic or anionic), although some of the emollients inherently possess emulsifying properties. These same general ingredients can be formulated into a cream rather than a lotion, or into gels, or into solid sticks by utilization of different proportions of the ingredients and/or by inclusion of thickening agents such as gums or other forms of hydrophillic colloids. Such compositions are referred to herein as cosmetically acceptable carriers. Preferably, the carrier should be a gel base formula without lipid materials that would exxacerbate the oiliness of acne prone skin. However, a moisturizer emulsion base may be preferred by individuals that have particularly dry yet skin still suffer from acne lesions.

The topical compositions according to the invention can comprise additional ingredients commonly found in skin care compositions, such as for example, emollients, skin conditioning agents, emulsifying agents, humectants, preservatives, antioxidants, perfumes, chelating agents, etc., provided that they are physically and chemically compatible with the other components of the composition. Noteably useful is the incorporation of vitamin A and vitamin A derivatives, including but not restricted to retinol, retinyl palmitate, retinoic acid, retinal, and retinyl propionate.

Examples of suitable preservatives for use in the compositions of the invention include the C₁-C₄ alkyl parabens and phenoxyethanol. Generally, the preservative is present in an amount ranging from about 0.5 to about 2.0, preferably about 1.0 to about 1.5, weight percent based on the total composition. In a preferred embodiment, the preservative is mixture of from about 0.2 to about 0.5 weight percent methylparaben, from about 0.2 to about 5.0 weight percent propylparaben and from about 0.05 to about 0.10 weight percent butylparaben. A particularly preferred commercially available preservative that may be used in the skin care composition according to this invention is PHENONIP TM which is a practically colorless, viscous, liquid mixture of phenoxyethanol, methylparaben, ethylparaben, propylparaben, and butylparaben available from Nipa Laboratories, Inc., Wilmington, Del.

Preferably, antioxidants should be present in the compositions according to the invention. Suitable antioxidants include butylated hydroxy toluene (BHT), ascorbyl palmitate, butylated hydroanisole (BHA), phenyl-α-naphthylamine, hydroquinone, propyl gallate, nordihydroquiaretic acid, vitamin E or derivatives of vitamin E, vitamin C and derivatives thereof, calcium pantothenic, green tea extracts and mixed polyphenosls, and mixtures thereof of the above. When utilized the antioxidant can be present in an amount ranging from about 0.02 to about 0.5% by weight, more preferably from about 0.002 to about 0.1% by weight of the total composition.

Emollients which can be included in the compositions of the invention function by their ability to remain on the skin surface or in the strarum comeum to act as lubricants, to reduce flaking, and to improve the skin appearance. Typical emollients include fatty esters, fatty alcohols, mineral oil, polyether siloxane copolymers and the like. Examples of suitable emollients include, but are not limited to, polypropylene glycol ("PPG")-15 stearyl ether, PPG-10 cetyl ether, steareth-10, oleth-8, PPG-4 lauryl ether, vitamin E acetate, PEG-7 glyceryl cocoate, lanolin, cetyl alcohol, octyl hydroxystearate, dimethicone, and combinations thereof. Cetyl alcohol, octyl hydroxystearate, dimethicone, and combinations thereof are preferred. When utilized, the emollient can be present in an amount from about 0.01 to about 5, preferably from about 1 to about 4 percent by weight based on the total composition.

Polyhydric alcohols can be utilized as humectants in the compositions of the invention. The humectants aid in increasing the effectiveness of the emollient, reduce scaling, stimulate removal of built-up scale and improve skin feel. Suitable polyhydric alcohols include, but are not limited to, glycerol (also known as glycerin), polyalkylene glycols, alkylene polyols and their derivatives, including butylene glycol, propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof. Glycerin is preferred. When utilized, the humectant is present in an amount from about 0.1 to about 5, preferably from about 1 to about 3 percent by weight, based on the total weight of the composition.

The compositions according to the invention preferably contain an effective stabilizing amount of an emulsifier. Preferably, the emulsifier is present at from about 1.0 to about 10.0, more preferably from about 3.0 to about 6.0, weight percent, based on the total composition. Any emulsifier that is compatible with the components of the composition can be employed. Suitable emulsifiers include stearic acid, cetyl alcohol, stearyl alcohol, steareth 2, steareth 20, Acrylates/C10-30 alkyl Acrylate Crosspolymer. Particularly preferred is PEMULEN TR-1 (CTFA Designation: Acrylates/10-30 Alkyl Acrylate Crosspolymer).

Any fragrance may be added to the compositions of the invention for aesthetic purposes. Suitable fragrances include, but are not limited to, eucalyptus oil, camphor synthetic, peppermint oil, dove oil, lavender, chamomile and the like. When utilized, fragrances are present in an amount from about 0.05 to about 0.5, preferably from about 0.1 to about 0.3 percent by weight, based on the total weight of the composition. In certain aspects of this invention, the compositions should include a chelaring agent. Chelating agents which are useful in the compositions of the present invention include ethylenediamine tetra acetic acid (EDTA) and derivatives and salts thereof, dihydroxyethyl glycine, tartaric acid, and mixtures thereof. The chelating agents should be utilized in a stabilizing effective amount and may range from about 0.01 to about 2% based on the weight of the total composition, preferably from about 0.05 to about 1%. Most preferably, the chelating agent should be EDTA.

Generally, the composition is topically applied to the affected skin areas in a predetermined or as-needed regimen to bring about improvement, it generally being the case that gradual improvement is noted with each successive application. Insofar as has been determined based upon clinical studies to date, no adverse side effects are encountered.

The advantages of the invention and specific embodiments of the skin care compositions prepared in accordance with the present invention are illustrated by the following examples. It will be understood, however, that the invention is not confined to the specific limitations set forth in the individual examples, but rather defined within the scope of the appended claims.

The following materials were used in the Example:
BRIJ 72: steareth 2 emulsifier commercially available from Uniqema.
BRIJ 721: steareth 20 emulsifier commercially available from Uniqema.
DIMETHICONE 47V-100: dimethicone 100 centistokes emollient commercially available form Rhodia.
EMERESSENCE 1160: phenoxyethanol commercially available from Cognis. FINSOLV TN: C₁₂-C₁₅ alkyl benzoate solubilizing agent commercially available from Finetex.
GLYPURE: 70% aqueous solution of glycolic acid commercially available from DuPont
WICKENOL 171: octyl hydroxystearate emollient commercially available from Alzo Inc.

### Example 1

The following formula was made in accordance with the teachings of this invention. The oil phase, water phase and tyrosine premix were formed separately. The tyrosine premix was prepared as follows: deionized water, DMAE, and tyrosine were added to a closed container and placed in a heated (50-55°C) water bath. The mixture was heated to about 50 to about 55°C. The mixture was held at that temperature with mixing until the water and oil phases were combined and cooled to about 45°C as described below.

The following oil phase ingredients (FINSOLV TN, WICKENOL 171, DIMETHICONE 47V-100, BRIJ 72, cetyl alcohol, BRIJ 721, and BHT) were blended together in a kettle and heated to about 60°C with agitation. Once the mixture was homogeneous PEMULEN was added with agitation until homogenous. The temperature was held at about 78-80°C until the water phase was prepared and ready for addition.

The water phase was prepared as follows: deionized water was added to a kettle and the kettle slowly heated to about 70 to about 75°C. During the heating process disodium EDTA, glycerin, panthenol (preheated until easy flowing liquid) were added. At about 78 to about 80°C propylparaben, methylparaben and phenoxyethanol were added The mixture was held at about 70 to about 75°C for about 3 to about 5 minutes, *i.e.,* until a uniform mixture was obtained.

When both phases were at a temperature of about 78 to about 80°C and homogenous, the oil phase was added to the water phase. The mixture was held at about 78 to about 80°C for about 10 to 15 minutes, *i.e.,* until a smooth, non-grainy dispersion was apparent. Heating was discontinued and when the temperature reached below 45°C the DMAE/Tyrosine premix followed by the buffer pre-mix were added and mixed well. The mixture was homogenized at 40% for about 3-4 minutes with a rotor-stator homogenizer.

| **INGREDIENT:** | **WEIGHT PERCENT:** |
|---|---|
| ***Water Phase:*** | |
| Deionized water | 62.69 |
| Disodium EDTA | 0.10 |
| Glycerin | 3.00 |
| Panthenol | 0.50 |
| EMERESSENCE 1160 | 0.73 |
| Methylparaben | 0.35 |
| Propylparaben | 0.17 |

| ***Oil Phase:*** | |
|---|---|
| FINSOLV TN | 4.00 |
| WICKENOL 171 | 1.00 |
| DIMETHICONE 47V-100 | 1.00 |
| BRIG 72 | 0.60 |
| Cetyl Alchol | 2.50 |
| BRIJ 721 | 0.90 |
| BHT | 0.10 |
| PEMULEN TR1 | 0.50 |

| ***DMAE/Tyrosine Premix:*** | |
|---|---|
| L-tyrosine | 0.50 |
| Deionized water | 15.00 |
| DMAE | 3.00 |

| ***Buffer Premix:*** | |
|---|---|
| GLYPURE 70 | 1.2 |
| Malic add | 0.84 |
| Deionized water | 1.32 |

### Example 2

The composition of Example 1 was tested its effects on subjects with mild acne a when topically applied to the skin to 29 subjects daily for a 45 day period. The subjects had the following demographics:

| No. of Subjects | Acne | Mean Age | Gender | Mean Weight |
|---|---|---|---|---|
| 29 | Mild* | 30 | Female | 157 lbs. |

| | | | | |
|---|---|---|---|---|
| *A subject with "mild acne" had greater than 10 non-inflammatory and inflammatory lesions at the start of the study, i.e., "baseline". | | | | |

The results are set forth in Figure 1. As can be seen from Figure 1, there was a significant decrease from baseline (p<0.001) in the average number of inflammatory papules in subjects with mild acne.

## Claims

1. A composition for topical application comprising an effective amount of an alkanolamine of the formula: 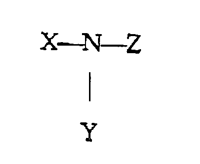 is cosmetically acceptable carrier, wherein X, Y and Z are selected from the group consisting of hydrogen, C₁-C₃ alkyl group, and C₂-C₄ alkanol group, wherein at least one of X, Y or Z is a C₂-C₄ alkanol group bearing at least one hydroxyl group and optionally at least one carboxyl group.

2. The composition of claim 1, wherein said alkanolamine is ethylaminoethanol, methylaminoethanol, dimethylaminoethanolamine, isopropanolamine, triethanolamine, isopropanoldimethylamine, ethylethanolamine, 2-butanolamine, choline or serine.

3. The composition of claim 2, wherein said alkanolamine is dimethylaminoethanol.

4. The composition of any one of claims 1 to 3, wherein said compound is present in an amount of from 0.1 to 10% by weight of the composition.

5. The composition of claim 4, wherein said compound is present in an amount of from 1 to 4% by weight of the composition.

6. The composition of any one of claims 1 to 5, wherein said composition further comprises tyrosine.

7. The composition of claim 6, wherein said tyrosine is present in an amount of from 0.01 to 5% by weight of the composition.

8. The composition of claim 7, wherein said tyrosine is present in an amount of from 0.04 to 3% by weight of the composition.

9. The composition of claim 8, wherein said tyrosine is present in an amount of from 0.04 to 0.5% by weight of the composition.

10. The composition of any one of claims 1 to 9, for use in reducing the number or severity of acne papules on mammalian skin.
